# EUROPEAN PATENT SPECIFICATION

(11) **EP 3 179 828 B1**
(45) Date of publication and mention of the grant of the patent: **22.04.2020**
(21) Application number: 17160456.4
(22) Date of filing: 12.03.2017
(51) Int. Cl.: H05B 3/42, A24F 47/00

(54) **CIGARETTE HEATING DEVICE**
ZIGARETTENERWÄRMUNGSVORRICHTUNG
DISPOSITIF DE CHAUFFAGE DE CIGARETTE

(30) Priority: 14.03.2016 CN 201620193316 U
(43) Date of publication of application: 14.06.2017
(73) Proprietor: Shenzhen First Union Technology Co., Ltd., Shenzhen, Guangdong 518104 (CN)
(72) Inventor: LI, Yonghai, Shenzhen, Guangdong 518104 (CN); XU, Zhongli, Shenzhen, Guangdong 518104 (CN); CHEN, Hongbin, Shenzhen, Guangdong 518104 (CN); ZHONG, Yunping, Shenzhen, Guangdong 518104 (CN); HUANG, Yanzhou, Shenzhen, Guangdong 518104 (CN)
(74) Representative: Proi World Intellectual Property GmbH

(56) References cited:
- EP-A1- 2 394 520
- US-A- 4 637 407
- US-A- 5 388 594
- US-A1- 2015 013 696
- US-A1- 2016 007 649

## Description

### TECHNICAL FIELD

The present invention relates to electronic cigarettes, and particularly to a cigarette heating device.

### BACKGROUND ART

Cigarette heating devices are configured for heating a cigarette to form aerosol. During this process, since the cigarette does not burn, a lot of cancerous material (e.g., tar) does not exist. Accordingly, the cigarette heating device is healthier for users. However, in a typical cigarette heating device, it is inconvenient to take the cigarette out after use. For example, US 2015/0013696 A1 discloses an electrically heated smoking system having an extractor, which includes a sliding receptacle for receiving the smoking article and a heater for heating the aerosol-forming substrate to form the aerosol.

US 2016/0007649 A1 discloses an inhalation device in which a coil spring is disposed within the cylindrical housing for biasing the ejection tube in a direction away from the annular wall.

US 4637407 A discloses a cigarette holder which uses a compression spring to urge the ejector and tip of the device to the left until the plunger head portion seats on the body portion.

EP 2394520 A1 discloses a tool for allowing smoker to inhale nicotine vaporized from tobacco leaves by directly heating the tobacco leaves without combusting the tobacco leaves.

US 5388594 A discloses a smoking system in which a replaceable cigarette containing tobacco flavor material is electrically heated by a set of electrical heater elements contained within a lighter.

However, all of the above prior art need to manually make the sliding receptacle move back to the initial first position after taking the cigarette out. This is very inconvenient for use.

What is needed, therefore, is a cigarette heating device, which can overcome the above shortcomings.

### SUMMARY

A cigarette heating device for heating a cigarette includes a main sleeve unit, a central heater, a movable element, an internal cup, and a restoring element. The main sleeve unit presets a first position and a second position along an axial direction. The movable element is movable between the first position and the second position. The internal cup is configured for receiving the cigarette. The internal cup is in linkage relation with the movable element. When the movable element is in the first position, the internal cup is adjacent to the central heater, so that the central heater heats the cigarette. When the movable element is in the second position, the internal cup is away from the central heater, so that the central heater is separated from the cigarette. The restoring element is configured for driving the movable element to restore from the second position to the first position.

### BRIEF DESCRIPTION OF THE DRAWINGS

Many aspects of the present disclosure can be better understood with reference to the following drawings. The components in the drawings are not necessarily drawn to scale, the emphasis instead being placed upon clearly illustrating the principles of the present disclosure. Moreover, in the drawings, like reference numerals designate corresponding parts throughout the several views.
FIG. 1 is a side view of a cigarette heating device including a movable element when the movable element is in a first position.
FIG. 2 is a partially cross-sectional view of the cigarette heating device of FIG. 1 when the movable element is in the first position.
FIG. 3 is a side view of the cigarette heating device when the movable element is in a second position, together with a cigarette.
FIG. 4 is a partially cross-sectional view of the cigarette heating device of FIG. 3 when the movable element is in the second position.

### DETAILED DESCRIPTION

Several definitions that apply throughout this disclosure will now be presented.

The term "outside" refers to a region that is beyond the outermost confines of a physical object. The term "inside" indicates that at least a portion of a region is partially contained within a boundary formed by the object. The term "substantially" is defined to be essentially conforming to the particular dimension, shape or other word that substantially modifies, such that the component need not be exact. For example, substantially cylindrical means that the object resembles a cylinder, but can have one or more deviations from a true cylinder. The term "comprising," when utilized, means "including, but not necessarily limited to"; it specifically indicates open-ended inclusion or membership in the so-described combination, group, series and the like.

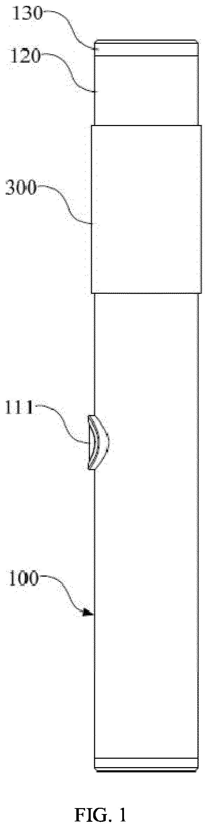

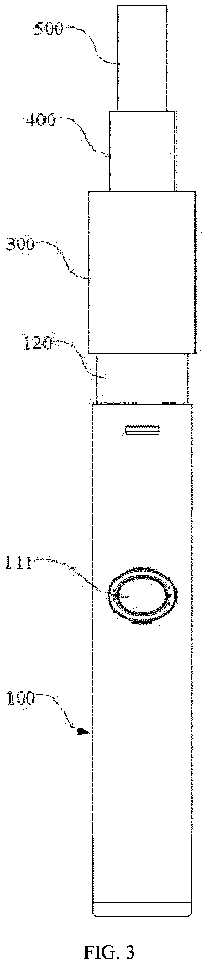

Referring to FIGS. 1-4, a cigarette heating device for heating a cigarette 500 is shown. The cigarette heating device includes a main sleeve unit 100, a central heater 200, a movable element 300, an internal cup 400, and a restoring element 600.

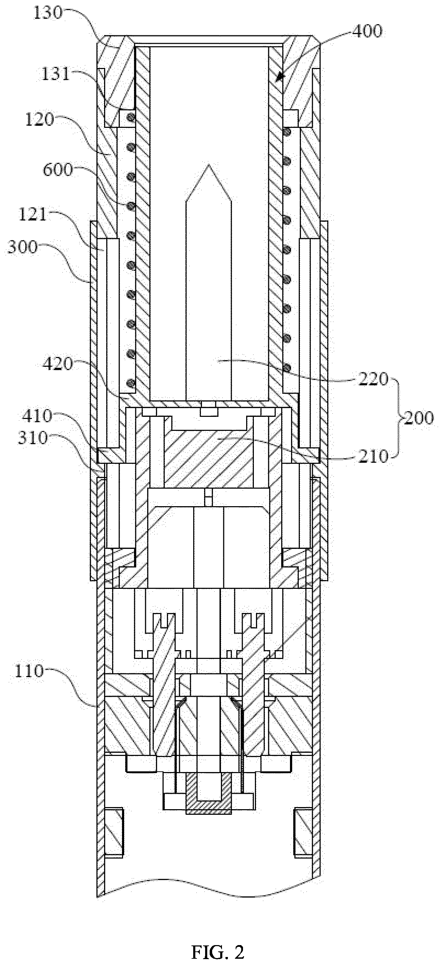

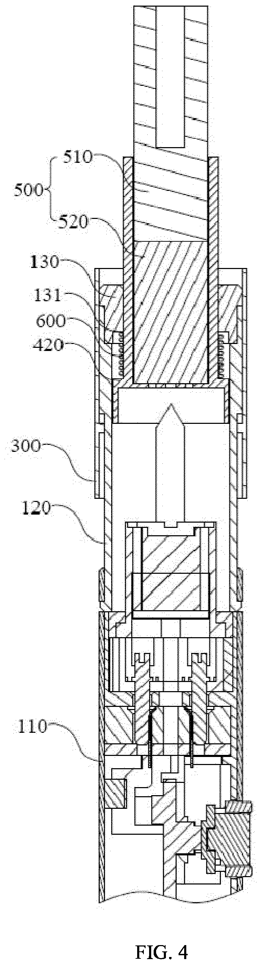

The main sleeve unit 100 presets a first position and a second position. The central heater 200 is arranged in the main sleeve unit 100. The movable element 300 is movable between the first position and the second position. The internal cup 400 is configured (i.e., structured and arranged) for receiving the cigarette 500, and moves together with the movable element 300. When the movable element 300 is in the first position (as seen in FIG. 2), the internal cup 400 is adjacent to the central heater 200, so that the central heater 200 can heat the cigarette 500; when the movable element 300 is in the second position (as seen in FIG. 4), the internal cup 400 is far away from the central heater 200, so that the central heater 200 is separated from the cigarette 500. The restoring element 600 is configured for driving the movable element 300 to restore from the second position to the first position.

To take out the cigarette 500, the movable element 300 is pushed/pulled upwards, the internal cup 400 and the cigarette 500 are then separated from a heating body 220 of the central heater 200. In this way, the cigarette 500 can be taken out conveniently. After being released, the internal cup 400 is driven to the first position by the restoring element 600.

In detail, in the present embodiment, the main sleeve unit 100 includes a battery tube 110, a heater tube 120, and a top engaging ring 130.

The battery tube 110 is configured for accommodating a battery, a printed circuit board and electronic components on the circuit board. A control button 111 is provided on the battery tube 110. In the drawings, only one control button 111 is shown.

The heater tube 120 is connected with the battery tube 110, and defines a groove 121. The central heater 200 is accommodated in the heater tube 120.

The top engaging ring 130 is connected to the heater tube 120, and the top engaging ring 130 includes a first stage 131.

The battery tube 110, the heater tube 120 and the top engaging ring 130 may be coupled by snap fit, or interference fit.

In the present embodiment, the central heater 200 includes a fixing holder 210 and a heating body 220 arranged on the fixing holder 210. The fixing holder 210 is fixedly connected with the battery tube 110. The heating body 220 includes a thermal conducting body and a heating wire formed on the thermal conducting body.

When the movable element 300 is in the first position (referring to FIG. 2), the internal cup 400 is adjacent to the fixing holder 210, so that the heating body 220 inserts the internal cup 400 via an opening at a bottom of the internal cup 400. When the movable element 300 is in the second position (referring to FIG. 4), the internal cup 400 is away from the fixing holder 210, such that the heating body 220 is moved out from the opening at the bottom of the internal cup 400.

The movable element 300 is arranged outside of the main sleeve unit 100, and the main sleeve unit 100 nests the internal cup 400. The movable element 300 includes a first linkage 310 on an inner surface, the heater tube120 defines a slot in a sidewall, and the internal cup 400 includes a second linkage 410. The first linkage 310 is engaged in the slot 121, and abuts against the second linkage 410. Alternatively, the first and the second linkages 310, 410 may be coupled by magnetic force, and the slot 121 may be omitted. That is, one of the first and the second linkages 310, 410 is a magnet, and the other of the first and the second linkages 310, 410 a magnet or a magnetic metal.

Quite usefully, in the present embodiment, the movable element 300 is a casing nesting the heater tube 120. It is to be understood that, in other embodiments, the movable element 300 may be an arc-shaped plate or a sliding piece.

The first and the second linkages 310, 410 are protrusions as shown in the drawings. It is noteworthy that, in other embodiments, the first linkage 310 may be a recess, and correspondingly, the second linkage 410 may be a long protrusion engaging in the recess. It is to be understood that, in other embodiments, the second linkage 410 may be a recess, and correspondingly, the first linkage 310 may be a long protrusion engaging in the recess. Alternatively, the second linkage 410 may be a bottom surface of the internal cup 400, and the first linkage 310 may be a long protrusion extending to the bottom surface of the internal cup 400, as long as the movable element 300 forms a linkage relation with the internal cup 400.

In the present embodiment, the slot 121 extends along an axial direction of the heater tube 120, and restricting at least one of the first linkage 310 and the second linkage 410. Referring to FIG. 2, the slot 121 restricts the first position of the first linkage 310; referring to FIG. 4, the slot 121 restricts the second position of the first linkage. The slot 121 has an opening at an end, so that at least one of the first and the second linkages can be engaged in the slot 121 by interference fit.

In the present embodiment, the main sleeve unit 100 includes the first stage 131, the internal cup 400 correspondingly includes a second stage 420, the restoring element 600 includes a spring, the spring nests between the main sleeve unit 100 and the internal cup 400, and two ends of the spring abut against the first stage 131 and the second stage 420, respectively.

In the present embodiment, the second linkage 410, the second stage 420, and an outer surface of the internal cup 400 are step shaped in sequence. In other embodiments, the second linkage 410 and the second stage 420 may be coplanar.

## Claims

1. A cigarette heating device for heating a cigarette, comprising:
a main sleeve unit (100) preset with a first position and a second position along an axial direction thereof;
a central heater (200) arranged in the main sleeve unit (100);
a movable element (300) movable between the first position and the second position along the axial direction of the main sleeve unit (100); and
an internal cup (400) adapted to receive a cigarette (500), the internal cup (400) being in linkage relation with the movable element (300), such that when the movable element (300) is in the first position, the internal cup (400) is adjacent to the central heater (200) so that the central heater (200) is able to heat the cigarette received in the internal cup; when the movable element (300) is in the second position, the internal cup (400) is away from the central heater (200) so that the central heater (200) is able to be separated from the cigarette received in the internal cup;
**characterized by** further comprising
a restoring element (600) configured for driving the movable element (300) to restore from the second position to the first position.

2. The cigarette heating device according to claim 1, wherein the movable element (300) is arranged outside of the main sleeve unit (100), the main sleeve unit (100) nests the internal cup (400), the movable element (300) comprises a first linkage (310) on an inner surface, the internal cup (400) has a second linkage (410), one of the first and the second linkages abuts against the other of the first and the second linkages via the main sleeve unit.

3. The cigarette heating device according to claim 2, wherein the main sleeve unit (100) defines a slot (121) in a sidewall, the slot extends along an axial direction of the main sleeve unit (100), and the slot restricts at least one of the first linkage (310) and the second linkage (410).

4. The cigarette heating device according to claim 3, wherein the main sleeve unit (100) comprises a first stage (131), the internal cup (400) correspondingly comprises a second stage (420), the restoring element (600) comprises a spring, the spring nests between the main sleeve unit (100) and the internal cup (400), and two ends of the spring abut against the first stage (131) and the second stage (420), respectively.

5. The cigarette heating device according to claim 1, wherein the central heater (200) comprises a fixing holder (210) and a heating body (220) arranged on the fixing holder; the internal cup (400) defines an opening in a bottom thereof; when the movable element (300) is in the first position, the internal cup (400) is adjacent to the fixing holder (210), so that the heating body (220) is inserted into the internal cup (400) via the opening; when the movable element (300) is in the second position, the internal cup (400) is away from the fixing holder (210), so that the heating body (220) is moved out of the internal cup (400) via the opening.

6. The cigarette heating device according to claim 4, wherein the main sleeve unit (100) comprises:
a battery tube (110);
a heater tube (120) connected with the battery tube, the slot (121) being defined by the heater tube (120), the central heater (200) being accommodated in the heater tube (120); and
a top engaging ring (130) connected to the heater tube (120), and the top engaging ring (130) comprising a first stage (131).

7. The cigarette heating device according to claim 6, wherein the slot (121) has an opening at an end, so that at least one of the first and the second linkages (310, 410) is engaged in the slot by interference fit.

8. The cigarette heating device according to claim 7, wherein each of the first and the second linkages (310, 410) is a protrusion.

9. The cigarette heating device according to claim 7, wherein the second linkage (410), the second stage (420), and an outer surface of the internal cup (400) are step shaped in sequence.

## Patentansprüche

1. Zigarettenheizvorrichtung zum Erhitzen einer Zigarette, umfassend:
eine Haupthülseneinheit (100), die eine erste Position und eine zweite Position entlang einer axialen Richtung davon voreinstellt;
eine Zentralheizung (200), die in der Haupthülseneinheit (200) angeordnet ist;
ein bewegliches Element (300), das zwischen der ersten Position und der zweiten Position entlang der axialen Richtung der Haupthülseneinheit (100) beweglich ist; und
ein interner Becher (400), die zum Aufnehmen der Zigarette (500) konfiguriert ist, wobei der interne Becher (400) in Verbindung mit dem beweglichen Element (300) steht, so dass, wenn sich das bewegliche Element (300) in der ersten Position befindet, sich der interne Becher (400) neben der zentralen Heizung (200) befindet, so dass die zentrale Heizung (200) in der Lage ist, die Zigarette zu erhitzen, die in dem internen Becher aufgenommen ist; wenn sich das bewegliche Element (300) in der zweiten Position befindet, ist der interne Becher (400) von der Zentralheizung (200) entfernt, so dass die Zentralheizung (200) von der Zigarette getrennt werden kann, die in dem internen Becher aufgenommen ist;
**gekennzeichnet durch**
ein Wiederherstellungselement (600), das konfiguriert ist, um das bewegliche Element (300) anzutreiben, um von der zweiten Position in die erste Position zurückzukehren.

2. Zigarettenheizvorrichtung nach Anspruch 1, wobei das bewegliche Element (300) außerhalb der Haupthülseneinheit (100) angeordnet ist, die Haupthülseneinheit (100) den inneren Becher (100) einnistet, das bewegliche Element (300) ein erstes Bindeglied (310) auf einer Innenfläche aufweist, der innere Becher (400) ein zweites Bindeglied (410) aufweist, wobei eines der ersten und zweiten Bindeglieder über die Haupthülseneinheit an der anderen dem ersten und dem zweiten Bindeglied anliegt.

3. Zigarettenheizvorrichtung nach Anspruch 2, wobei die Haupthülseneinheit (100) einen Schlitz (121) in einer Seitenwand definiert, sich der Schlitz entlang einer axialen Richtung der Haupthülseneinheit (100) erstreckt und der Schlitz mindestens eine der ersten und der zweiten Bindeglieder einschränkt.

4. Zigarettenheizvorrichtung nach Anspruch 3, wobei die Haupthülseneinheit (100) eine erste Stufe (131) umfasst, der innere Becher (400) entsprechend eine zweite Stufe (420) umfasst, das Rückstellelement (600) eine Feder umfasst, die Feder zwischen der Haupthülseneinheit (100) und dem inneren Becher (400) einnistet, und zwei Enden der Feder an die erste Stufe (131) bzw. die zweite Stufe (420) anstößt.

5. Zigarettenheizvorrichtung nach Anspruch 1, wobei die Zentralheizung (200) einen Fixierhalter (210) und einen auf dem Fixierhalter (210) angeordneten Heizkörper (220) umfasst; der innere Becher (400) definiert eine Öffnung in einem Boden davon; wenn sich das bewegliche Element (300) in der ersten Position befindet, befindet sich der innere Becher (400) neben dem Befestigungshalter (210), so dass der Heizkörper (220) über die Öffnung in den inneren Becher (400) eingeführt wird; wenn sich das bewegliche Element (300) in der zweiten Position befindet, ist der innere Becher (400) vom Befestigungshalter (210) entfernt, so dass sich der Heizkörper (220) über die Öffnung aus dem inneren Becher (400) herausbewegt.

6. Zigarettenheizvorrichtung nach Anspruch 4, wobei die Haupthülseneinheit (100) umfasst:
eine Batterieröhre (110);
ein Heizungsrohr (120), das mit dem Batterierohr verbunden ist, wobei der Schlitz (121) mittels des Heizungsrohres (120) definiert ist, wobei die zentrale Heizung (200) in dem Heizungsrohr (120) untergebracht ist; und
einen oberen Eingriffsring (130), der mit dem Heizrohr (120) verbunden ist, und der obere Eingriffsring (130) eine erste Stufe (131) umfasst.

7. Zigarettenheizvorrichtung nach Anspruch 6, wobei der Schlitz (121) an einem Ende eine Öffnung aufweist, so dass mindestens eines der ersten und zweiten Bindeglieder (310,410) durch Presspassung in den Schlitz eingreift.

8. Zigarettenheizvorrichtung nach Anspruch 7, wobei jede der ersten und zweiten Bindeglieder (310,410) ein Vorsprung ist.

9. Zigarettenheizvorrichtung nach Anspruch 7, wobei das zweite Bindeglied (410), die zweite Stufe (420) und eine Außenfläche des inneren Bechers (400) nacheinander stufenförmig sind.

## Revendications

1. Dispositif chauffant de cigarette destiné au chauffage d'une cigarette, comprenant :
une unité de manchon principal (100) préréglée comportant une première position et une seconde position le long d'une direction axiale de celle-ci ;
un élément chauffant central (200) agencé dans l'unité de manchon principal (100) ;
un élément mobile (300) mobile entre la première position et la seconde position le long de la direction axiale de l'unité de manchon principal (100) ; et
une coupelle intérieure (400) adaptée à recevoir une cigarette (500), la coupelle intérieure (400) étant en relation de liaison avec l'élément mobile (300), de sorte que lorsque l'élément mobile (300) est dans la première position, la coupelle intérieure (400) est adjacente à l'élément chauffant central (200) de manière à ce que l'élément chauffant central (200) soit en mesure de chauffer la cigarette reçue dans la coupelle intérieure ; lorsque l'élément mobile (300) est dans la seconde position, la coupelle intérieure (400) est éloignée de l'élément chauffant central (200) de manière à ce que l'élément chauffant central (200) soit en mesure d'être séparé de la cigarette reçue dans la coupelle intérieure ;
**caractérisé par** comprenant en outre
un élément de rappel (600) conçu pour conduire l'élément mobile (300) pour revenir depuis la seconde position à la première position.

2. Dispositif chauffant de cigarette selon la revendication 1, dans lequel l'élément mobile (300) est agencé à l'extérieur de l'unité de manchon principal (100), l'unité de manchon principal (100) héberge la coupelle intérieure (400), l'élément mobile (300) comprend une première liaison (310) sur une surface intérieure, la coupelle intérieure (400) présente une seconde liaison (410), une des première et seconde liaisons vient en butée contre l'autre des première et seconde liaisons par l'intermédiaire de l'unité de manchon principal.

3. Dispositif chauffant de cigarette selon la revendication 2, dans lequel l'unité de manchon principal (100) définit une rainure (121) dans une paroi latérale, la rainure s'étend le long d'une direction axiale de l'unité de manchon principal (100) et la rainure restreint au moins une parmi la première liaison (310) et la seconde liaison (410).

4. Dispositif chauffant de cigarette selon la revendication 3, dans lequel l'unité de manchon principal (100) comprend un premier étage (131), la coupelle intérieure (400) comprend de manière correspondante un second étage (420), l'élément de rappel (600) comprend un ressort, le ressort niche entre l'unité de manchon principal (100) et la coupelle intérieure (400) et deux extrémités du ressort viennent en butée contre le premier étage (131) et le second étage (420), respectivement.

5. Dispositif chauffant de cigarette selon la revendication 1, dans lequel l'élément chauffant central (200) comprend un support de fixation (210) et un corps chauffant (220) agencés sur le support de fixation ; la coupelle intérieure (400) définit une ouverture dans une partie inférieure de celui-ci ; lorsque l'élément mobile (300) est dans la première position, la coupelle intérieure (400) est adjacente au support de fixation (210), de sorte que le corps chauffant (220) est inséré dans la coupelle intérieure (400) par l'intermédiaire de l'ouverture ; lorsque l'élément mobile (300) est dans la seconde position, la coupelle intérieure (400) est éloignée du support de fixation (210), de manière à ce que le corps chauffant (220) soit déplacé hors de la coupelle intérieure (400) par l'intermédiaire de l'ouverture.

6. Dispositif chauffant de cigarette selon la revendication 4, dans lequel l'unité de manchon principal (100) comprend :
un tube de batterie (110) ;
un tube d'élément chauffant (120) relié au tube de batterie, la rainure (121) étant définie par le tube d'élément chauffant (120), l'élément chauffant central (200) étant logé dans le tube d'élément chauffant (120) ; et
une bague d'entrée en prise supérieure (130) reliée au tube d'élément chauffant (120) la bague d'entrée en prise inférieure (130) comprenant un premier étage (131).

7. Dispositif chauffant de cigarette selon la revendication 6, dans lequel la rainure (121) présente une ouverture à une extrémité, de manière à ce qu'au moins une parmi les première et seconde liaisons (310, 410) soit en prise dans la rainure par ajustement serré.

8. Dispositif chauffant de cigarette selon la revendication 7, dans lequel chacune des première et seconde liaisons (310, 410) est une partie saillante.

9. Dispositif chauffant de cigarette selon la revendication 7, dans lequel la seconde liaison (410), le second étage (420) et une surface extérieure de la coupelle intérieure (400) sont en forme de marche dans cet ordre.
